Europäisches Patentamt

European Patent Office

Office européen des brevets

(11) Publication number: **0 187 714**
**B1**

(12) **EUROPEAN PATENT SPECIFICATION**

(45) Date of publication of patent specification: **26.09.90**

(51) Int. Cl.⁵: **C 07 C 51/44,** C 07 C 53/124

(21) Application number: **86300090.7**

(22) Date of filing: **08.01.86**

(54) **Process for separating components of mixtures comprising sulfuric acid and carboxylic acid.**

(30) Priority: **09.01.85 US 690076**
**19.12.85 US 809323**

(43) Date of publication of application:
**16.07.86 Bulletin 86/29**

(45) Publication of the grant of the patent:
**26.09.90 Bulletin 90/39**

(84) Designated Contracting States:
**AT BE CH DE FR GB IT LI LU NL SE**

(56) References cited:
**FR-A-2 135 729**
**GB-A-1 016 087**
**US-A-3 658 895**

(73) Proprietor: **ROHM AND HAAS COMPANY**
**Independence Mall West**
**Philadelphia Pennsylvania 19105 (US)**

(72) Inventor: **Davies, Philip David Trevor**
**531 Misty Lake Drive**
**Seabrook Texas 77586 (US)**
Inventor: **Kirch, Lawrence Samuel**
**871 Oriole Lane**
**Huntingdon Valley, Pa. 19006 (US)**

(74) Representative: **Angell, David Whilton et al**
**ROHM AND HAAS (UK) LTD. European**
**Operations Patent Department Lennig House 2**
**Mason's Avenue**
**Croydon CR9 3NB (GB)**

Courier Press, Leamington Spa, England.

**Description**

This invention is concerned with separating components of a mixture comprising sulfuric acid and carboxylic acid such as that produced from a sulfuric acid-catalyzed carbonylation of olefinic compound(s).

In commercial processes it is important to obtain a clean separation of the components of a product mixture for efficient recycle of one or more of the components and for recovery of useful products in high yields. In addition, careful attention must be given to costs of energy, such as may be required in distillation or other separation techniques, in order to make the overall process economical and competitive.

Illustrative of a process to which the foregoing considerations are applicable is the separation of sulfuric acid (for recycle) and alkanoic acid (as product) from the reaction product mixture of a sulfuric acid-catalyzed carbonylation of olefins (Koch process-U.S. Patent No. 2,831,877). The carboxylic acids resulting from the process can be used for a wide variety of purposes but are particularly useful as the feed in large scale oxydehydrogenation processes for the production of unsaturated carboxylic acids.

Sulfuric acid and carboxylic acid have been separated by vacuum distillation such as described in U.S. Patents Nos. 3,632,638 (Hyman) and 3,663,613 (Pai and Hyman). In this method the addition of water to the feed mixture in the distillation column is known to promote the separation of the acids.

Moreover, although a large amount of dilution water will prevent decomposition of the carboxylic acid product by reversing the protonation of the carboxylic acid by the sulfuric acid, the high water content in the distillation pot will also reduce the boiling point of the sulfuric acid/carboxylic acid feed and thereby limit the distillation pot temperature to about 90°C (35 mm Hg vacuum pressure). The separation, therefore, is not as good as it could be if higher distillation temperatures were possible. In addition, even after the water reverses the protonation of the carboxylic acid by the sulfuric acid, the carboxylic acid remains strongly bound to the sulfuric acid by hydrogen bonding. The large amount of water present in the pot not only makes it difficult to supply enough energy to break these bonds but, also because there is little boil-up from the pot and no reflux, any carboxylic acid reaching the sulfuric acid rich pot will become trapped. The hydrogen bonds therefore cannot be broken merely by supplying more heat to the pot.

A combination of solvent extraction and distillation provides good separation of the components of a sulfuric acid/carboxylic acid mixture but again a large amount of dilution water is required which must be boiled off overhead, and the solvent must be removed.

We have now found a method for separating sulfuric acid and carboxylic acid, for example, the separation of such components from the reaction product mixture obtained from the sulfuric acid-catalyzed carbonylation of olefins to carboxylic acids according to the Koch synthesis, which enables such separation to be carried out in a more effective, less energy intensive and therefore more economical manner, for example, by minimizing the amount of water which is required to be added to a mixture comprising sulfuric acid and carboxylic acid during separation thereof with consequent savings in the cost of removing the water.

According to the present invention there is provided a method of separating components of a mixture comprising sulfuric acid and carboxylic acid, the method comprising feeding the mixture or components thereof, to a distillation column, diluting the mixture while in the column with sufficient water to provide a distillation mixture having a water:sulfuric acid mole ratio of 1.09:1 to 1.5:1, and distilling the mixture. For purposes of further discussion the foregoing dilution technique is hereinafter sometimes described as "internal dilution" as contrasted with the "external dilution" of prior practice as described in the Hyman and Pai/Hyman patents identified above.

Among the advantages achieved by the internal dilution is the use of the large amount of energy released during the internal dilution to raise the temperature within the column. Consequently, the distillation does not depend solely on pot temperature, and the pot may be operated at higher than normal temperatures. These conditions combine to provide a very hot water-rich region within the column which causes substantially complete separation of the sulfuric and carboxylic acids using a minimum amount of water. In large scale processes this translates into very substantial energy savings due to the lower amounts of water which must be boiled off for recovery of concentrated sulfuric acid. For example, the dilution of a mixture of 85 weight percent sulfuric acid and 15 weight percent isobutyric acid to 65 weight percent sulfuric acid requires 2.04 kg water per kg isobutyric acid but dilution to 72.5 weight percent sulfuric acid requires only 1.15 kg water per kg isobutyric acid, thus saving 0.89 kg water. The amount of energy required to boil off this excess amount of water will be enormous on an industrial scale.

The separation method of the invention is practiced on a mixture comprising sulfuric acid and carboxylic acid, whether such mixture is formed externally of the distillation column and then fed to the column, or whether the components are fed separately to the column for admixture in the column. A preferred industrial application is the separation of the components of an effluent product stream from the sulfuric acid-catalyzed carbonylation of olefin. The sulfuric acid for the carbonylation is concentrated, i.e., at least 90%, preferably at least 96%. By careful carboxylation the water may be eliminated or reduced to less than 5 weight percent.

The feed mixture used in the method of the present invention, for example the product stream from the sulfuric acid-catalyzed carbonylation of olefin or a mixture formed within the column from separate feeds of sulfuric acid and carboxylic acid comprises from 50 to 90 weight percent of sulfuric acid (100% basis), 10 to 50 weight percent carboxylic acid and 0 to 5 weight percent water. For example, the feed mixture may

EP 0 187 714 B1

comprise 75 to 85 weight percent sulfuric acid, 15 to 25 weight percent carboxylic acid, and 0 to 4 weight percent water.

The carboxylic acids are low boiling, that is, have a boiling point lower than that of sulfuric acid, and may contain from 2 to about 30 carbon atoms, for example, depending upon the olefins from which they may be derived. Typical olefinic precursor compounds are aliphatic olefins, such as ethylene, propylene, butylene, and isobutylene, or higher molecular weight olefins such as nonene and hexadecene, including mixtures thereof; cyclic olefins such as cyclohexene; aliphatic or alicyclic diolefins such as butadiene and 4-vinylcyclohexene-1; other polyolefinic compounds which lead to unsaturated carboxylic acids such as oleic acid; and olefinic compounds containing other functional groups, such as 1,2-dichloroethylene and cinnamyl alcohol. The corresponding carboxylic acids include propionic acid, isobutyric acid and trimethylacetic acid, such as are disclosed in U.S. Patents Nos. 2,831,877 and 3,282,973. Other carboxylic acids, not synthesized directly by the Koch process, are also separable from sulfuric acid by the process of the invention. These include phthalic acid, acrylic acid and methacrylic acid. The invention is also applicable to mixtures of carboxylic acids.

The mixture comprising sulfuric acid, carboxylic acid and water (if any) is conveniently distilled in a conventional distillation column equipped with heating mantle, agitation means, bottoms receiver, condenser, overhead receiver and other known controls and elements. The distillation is preferably operated under vacuum distillation conditions, for example, at 3,333 to 13,330 Pa (25 to 100 mm) preferably 3,999 to 6,665 Pa (30 to 50 mm), and more preferably, 4,666 to 5,999 (35 to 45) mm Hg pressure. The distillation temperature may range from the boiling point of the carboxylic acid to below its decomposition temperature. At a distillation pressure of 4,666 Pa (35 mm Hg) for the distillation of a mixture comprising sulfuric acid and isobutyric acid, this temperature will be in the range of from 100° to 160°C in a continuous distillation and not over about 120°C for batch distillations. These conditions will vary, of course, with the carboxylic acid to be separated from the sulfuric acid.

One of the principle advantages of the invention is the ability to operate at the higher temperatures due to the large quantity of energy released upon the internal dilution, thus facilitating the separation. In the known external dilution separation processes, it is difficult to operate at temperatures higher than about 90°C because the high water content of the feed mixture limits the pot temperature to about 90°C. Continuous, thin film vacuum distillation or distillation in an Oldershaw column are suitable distillation techniques. Residence time in the continuous distillation will depend on the continuous distillation as well as on the proportions of components in the distillation mixture and other operating conditions. Generally for a continuous distillation at a temperature in the range from about 120° to 160°C, 4,666 Pa (35 mm Hg) pressure and a mixture comprising sulfuric acid and isobutyric acid, a bottoms residence time of from about 10 to 40 minutes will be sufficient, but this will vary depending on the carboxylic acid in the mixture.

The dilution water is fed to the column at a point below the point of entry of the feed mixture or the components thereof. The improvement in separation achieved in this manner is believed to be a consequence of greater interaction between the sulfuric acid and water resulting from reduced opportunity for the water to flash through the sulfuric acid. For example, in a 20-plate Oldershaw column, if the sulfuric alone or a mixture comprising sulfuric acid and carboxylic acid is fed at trays 12 to 17, the dilution water is preferably fed at about tray 10 or lower.

The amount of dilution water is such as to provide a water:sulfuric acid mole ratio of 1.09:1 to 1.5:1, in order to minimize the amount of water which must be removed. The process generally will provide 80 to 90% sulfuric acid which can be further concentrated by subsequent distillation, if desired.

In one embodiment of the invention, wherein the carboxylic acid is isobutyric acid, water is added sufficient to provide 65 to 75 weight percent sulfuric acid, 15 to 25 weight percent isobutyric acid, and 10 to 15 weight percent total water in the distillation mixture at the outset of distillation.

Other conditions and aspects of the separation method are conventional and will be readily apparent to those skilled in the art. For example, it may be advantageous to utilize inert gas (e.g. nitrogen) stripping in conjunction with the distillation, or to recycle product to the same or other distillation apparatus for additional separation.

The present invention will now be further illustrated by way of the following Examples which are for illustrative purposes only and are not to be construed as imposing any limitation on the scope of the invention. All parts and percentages are by weight unless otherwise stated.

## EXAMPLES
### Run Nos. 1 and 2 — Internal Dilution

From the carbonylation of propylene with concentrated sulfuric acid and carbon monoxide at a mole ratio of 1:4:3 and pressure above 10, 13, M Pa (100 atmospheres), there was obtained a product mixture containing 15% by weight isobutyric acid (IBA, selectivity 95%) and 85% by weight sulfuric acid. This reactor effluent was fed to a distillation apparatus consisting of a stirred bottoms vessel with overflow level control, a 20-plate Oldershaw column, and a water-cooled condenser. Feeding of the carbonylation effluent was to tray 12 of the Oldershaw column. Enough water to achieve a 1.34:1 molar ratio with the sulfuric acid was fed to tray 10 of the column. The bottoms pot was maintained at 150° to 160°C and the residence time of the material therein was about 25 minutes. The distillation was at a pressure of 4,666 Pa (35 mm Hg) and resulted in recovery of 99% of the IBA overhead and a bottoms concentration of 85% sulfuric acid. Further

3

# EP 0 187 714 B1

concentration of the sulfuric acid by distillation produced a catalyst suitable for recycle to the carbonylation process. The conditions and results of this experiment are summarized as Run 2 of Table I below.

External Dilution

In contrast, when the carbonylation product effluent was diluted prior to feeding to the distillation column, to obtain 70% by weight sulfuric acid in the distillation mixture, the bottoms stream contained 1.7% by weight IBA which charred considerably to give a pitch black color upon concentration. In this distillation the material boiled at 90°C [4,666 Pa (35mm Hg.)]. This result is summarized as Run 1 of Table I below.

Table I summarizes the results of the foregoing Runs and other runs wherein it is shown that feeding the carbonylation product to the column at a still higher level, i.e., at tray 17 rather than tray 12 as in Runs 4, 7 and 10, provided additional improvement. This improvement is particularly evident from Runs 3 and 4. Run 3, although operated at a close to 1:1 mole ratio of sulfuric acid to IBA, resulted in 3.4% IBA in the bottoms streams and thus is indicated as unacceptable result ("acceptable" means about 97 to 99% IBA recovery; "unacceptable" is less than 97% IBA recovery). The IBA recovery was improved by the greater spacing of the feed trays in Run 4. Runs 5, 6, 8 and 10 show distillation wherein the water:sulfuric acid mole ratios were less than 1:1 and therefore provided poorer separations. Runs 2, 4, 7 and 9 are representative of the process of the invention; the remaining runs show other, disadvantageous separation conditions.

4

TABLE I

| Run No. | Carbonylation Product H₂SO₄ | Feed IBA | Overall Dilution H₂SO₄ | IBA | Water | Internal Dilution | Mole Ratio H₂SO₄:H₂O | Carbonylation Product Feed Tray | Water Feed Tray | Separation |
|---|---|---|---|---|---|---|---|---|---|---|
| | % | % | % | % | % | | | | | |
| 1. | 82.5 | 17.5 | 69.8 | 14.9 | 15.3 | No | 1:1.19 | 12 | -- | Unacceptable |
| 2. | 85 | 15 | 70.3 | 12.4 | 17.3 | Yes | 1:1.34 | 12 | | Acceptable |
| 3. | 85 | 15 | 72.9 | 12.8 | 14.3 | Yes | 1:1.07 | 12 | 10 | Unacceptable |
| 4. | 85 | 15 | 72.7 | 12.8 | 14.5 | Yes | 1:1.09 | 17 | 10 | Acceptable |
| 5. | 85 | 15 | 75.5 | 13.2 | 11.8 | Yes | 1:0.86 | 17 | 10 | Unacceptable |
| 6. | 85 | 15 | 75.0 | 13.2 | 11.8 | Yes | 1:0.86 | 17 | 4 | Unacceptable |
| 7. | 80 | 20 | 68.8 | 17.2 | 14.0 | Yes | 1:1.11 | 17 | 10 | Acceptable |
| 8. | 80 | 20 | 70.7 | 17.7 | 11.6 | Yes | 1:0.89 | 17 | 10 | Unacceptable |
| 9. | 75 | 25 | 64.9 | 21.6 | 13.5 | Yes | 1:1.13 | 17 | 10 | Acceptable |
| 10. | 75 | 25 | 67.0 | 22.3 | 10.7 | Yes | 1:0.87 | 17 | 10 | Unacceptable |

EP 0 187 714 B1

## EP 0 187 714 B1

### Claims

1. A method of separating components of a mixture comprising 50 to 90 weight percent sulfuric acid, 10 to 50 weight percent carboxylic acid having a boiling point lower than that of sulfuric acid, and 0 to 5 weight percent water, the method comprising feeding the mixture or the components thereof into a distillation column, diluting the mixture while in the column with sufficient water to provide a distillation mixture having a water:sulfuric acid mole ratio of 1.09:1 to 1.5:1, the dilution water being fed into the column below the point of entry of the mixture or the components thereof, and distilling the resulting mixture under vacuum.

2. A method as claimed in claim 1 wherein the feed mixture comprises 75 to 85 weight percent sulfuric acid, 15 to 25 weight percent carboxylic acid, and 0 to 4 weight percent water, and the amount of dilution water is sufficient to provide 10 to 20 weight percent total water in the distillation mixture at the outset of the distillation.

3. A method as claimed in any of claims 1 or 2 wherein the carboxylic acid is isobutyric acid.

4. A method as claimed in claim 3 wherein the water is sufficient to provide in the distillation mixture at the outset of the distillation, 65 to 75 weight percent sulfuric acid, 15 to 25 weight percent isobutyric acid, and 10 to 15 weight percent total water.

5. A method as claimed in any of claims 1 to 4 wherein the distillation is conducted in a multiplate column, the feed mixture is fed to an upper tray and the dilution water is fed to a tray below said upper tray.

6. A method as claimed in claim 5 wherein the distillation temperature is from 100°C to 160°C at a pressure of 3,999 to 6,665 Pa (30mm to 50mm Hg).

7. A method as claimed in any of claims 1 to 6 wherein the feed mixture is an effluent stream from a sulfuric acid-catalyzed carbonylation of an olefinic compound(s).

8. A method as claimed in claim 7 wherein the olefinic compound(s) is propylene.

9. A method as claimed in claim 7 or 8 wherein the carboxylic acid is isobutyric acid.

### Patentansprüche

1. Verfahren zur Trennung von Komponenten einer Mischung aus 50 bis 90 Gew.-% Schwefelsäure, 10 bis 50 Gew.-% Carbonsäure mit einem Siedepunkt unterhalb demjenigen der Schwefelsäure und 0 bis 50 Gew.-% Wasser, dadurch gekennzeichnet, daß die Mischung oder ihre Komponenten in eine Destillationskolonne eingeführt werden, die Mischung in der Kolonne mit einer solchen Menge Wasser verdünnt wird, die dazu ausreicht, eine Destillationsmischung mit einem Molverhältnis Wasser:Schwefelsäure von 1,09:1 bis 1,5:1 zu ergeben, das Verdünnungswasser in die Säule unterhalb des Eintrittspunkts der Mischung oder ihre Komponenten eingeführt wird und die erhaltene Mischung unter Vakuum destilliert wird.

2. Verfahren nach Anspruch 1, wobei die Beschickungsmischung 75 bis 85 Gew.-% Schwefelsäure, 15 bis 25 Gew.-% Carbonsäure und 0 bis 4 Gew.-% Wasser enthält und die Menge an Verdünnungswasser dazu ausreicht, 10 bis 20 Gew.-% Gesamtwasser in der Destillationsmischung am Ende der Destillation zu ergeben.

3. Verfahren nach einem der Ansprüche 1 oder 2, wobei die Carbonsäure aus Isobuttersäure besteht.

4. Verfahren nach Anspruch 3, wobei das Wasser dazu ausreicht, am Ausgang der Destillation in der Destillationsmischung 65 bis 75 Gew.-% Schwefelsäure, 15 bis 25 Gew.-% Buttersäure und 10 bis 15 Gew.-% Gesamtwasser einzustellen.

5. Verfahren nach einem der Ansprüche 1 bis 4, wobei die Destillation in einer aus mehreren Böden bestehenden Kolonne durchgeführt wird, die Beschickungsmischung einem oberen Boden zugeleitet wird und das Verdünnungswasser einem Boden unterhalb des oberen Bodens zugeleitet wird.

6. Verfahren nach Anspruch 5, wobei die Destillationstemperatur 100 bis 160°C bei einem Druck von 3999 bis 6665 Pa (30 bis 50 mm Hg) beträgt.

7. Verfahren nach einem der Ansprüche 1 bis 6, wobei die Beschickungsmischung ein Ablaufstrom einer Schwefelsäure katalysierten Carbonylierung einer oder mehrerer olefinischer Verbindungen ist.

8. Verfahren nach Anspruch 7, wobei die olefinische Verbindung oder die olefinischen Verbindungen aus Propylen bestehen.

9. Verfahren nach Anspruch 7 oder 8, wobei die Carbonsäure aus Isobuttersäure besteht.

### Revendications

1. Procédé de séparation des composants d'un mélange comprenant 50 à 90% en poids d'acide sulfurique, 10 à 50% en poids d'acide carboxylique ayant un point d'ébullition inférieur à celui de l'acide sulfurique, et 0 à 5% en poids d'eau, le procédé comprenant l'alimentation du mélange ou des composants de celui-ci dans une colonne de distillation, la dilution du mélange alors qu'il est dans la colonne avec suffisament d'eau pour fournir un mélange de distillation ayant un rapport molaire eau:acide sulfurique de 1,09:1 à 1,5:1, l'eau de dilution étant alimentée dans la colonne au-dessous du point d'entrée du mélange ou des composants de celui-ci, et la distillation du mélange résultant sous vide.

2. Procédé selon la revendication 1, dans lequel le mélange d'alimentation comprend 75 à 85% en poids d'acide sulfurique, 15 à 25% en poids d'acide carboxylique, et 0 à 4% en poids d'eau, et la quantité

6

d'eau de dilution est suffisante pour fournir 10 à 20% en poids d'eau totale dans le mélange de distillation à la sortie de la colonne de distillation.

3. Procédé selon l'une quelconque des revendications 1 ou 2, dans lequel l'acide carboxylique est l'acide isobutyrique.

4. Procédé selon la revendication 3, dans lequel l'eau est suffisante pour fournir dans le mélange de distillation à la sortie de la distillation, 65 à 75% en poids d'acide sulfurique, 15 à 25% en poids d'acide isobutyrique, et 10 à 15% en poids d'eau totale.

5. Procédé selon l'une quelconque des revendications 1 à 4, dans lequel la distillation est effectuée dans une colonne multiplateaux, le mélange d'alimentation est alimenté sur un plateau supérieur et l'eau de dilution est alimentée sur un plateau au-dessous dudit plateau supérieur.

6. Procédé selon la revendication 5, dans lequel la température de distillation est de 100°C à 160°C à une pression de 3.999 à 6.665 Pa (30 mm à 50 mm Hg).

7. Procédé selon l'une quelconque des revendications 1 à 6, dans lequel le mélange d'alimentation est un courant d'effluent provenant d'une carbonylation d'un ou de plusieurs composé(s) oléfinique(s), catalysée par l'acide sulfurique.

8. Procédé selon la revendication 7, dans lequel le ou les composé(s) oléfinique(s) est le propylène.

9. Procédé selon la revendication 7 ou 8, dans lequel l'acide carboxylique est l'acide isobutyrique.